# EUROPEAN PATENT SPECIFICATION

(11) **EP 2 732 260 B1**
(45) Date of publication and mention of the grant of the patent: **14.09.2016**
(21) Application number: 12814162.9
(22) Date of filing: 16.07.2012
(51) Int. Cl.: G01N 1/02, A61B 10/00

(54) **SAMPLE COLLECTION KIT**
PROBENSAMMELKIT
KIT DE PRÉLÈVEMENT D'ÉCHANTILLON

(30) Priority: 15.07.2011 US 201161508299 P
(43) Date of publication of application: 21.05.2014
(73) Proprietor: OraSure Technologies, Inc., Bethlehem, PA 18015 (US)
(72) Inventor: NONNEMACHER, Sheena, Bethlehem, PA 18018 (US); BLUM, Kristen, Whitehall, PA 18052 (US); FRITCH, Dean, East Greenville, PA 18041 (US); SAVARD, Mike, Macungie, PA 18062 (US); KARDOS, Keith, Bethlehem, PA 18015 (US); MURPHY, Brian, Baltimore, MD 21224 (US); SAVAGE, Jeremy, Baltimore, MD 21230 (US); ASKIN, Erik, Baltimore, MD 21224 (US)
(74) Representative: Jacobi, Markus Alexander
(86) International application number: PCT/US2012/046879
(87) International publication number: WO 2013/012785

(56) References cited:
- CN-A- 101 039 683
- CN-A- 101 528 763
- US-A- 5 103 836
- US-A- 5 609 160
- US-A1- 2002 115 089
- US-A1- 2004 082 878
- US-A1- 2008 118 397
- US-A1- 2008 241 001
- US-A1- 2009 024 060
- US-A1- 2009 170 072
- US-A1- 2010 150 778
- US-B2- 6 840 911
- US-B2- 7 238 519

## Description

### TECHNICAL FIELD

The invention relates generally to a method and kit for collecting samples of bodily fluids. More particularly, the invention also relates to an oral-fluid collecting device and kit for collection and testing of the fluid.

### BACKGROUND

Humans produce up to 1.5 liters of saliva each day. The use of saliva or "oral fluid" samples is well established for substance of abuse or drug testing and disease testing. Collecting oral fluid specimens is generally considered to be less invasive and less embarrassing, and less stigmatizing than the collection of other bodily fluids, such as blood, serum, urine, etc. The term "oral fluid" is generally considered a better descriptor than "saliva" for the fluid collected in oral specimens. Oral fluids are produced from multiple glands in the mouth. Oral fluid is made up of both saliva and mucosal excretions. Oral fluids contain glandular and cellular debris present in the oral cavity as well as components of blood which include antibodies and drug metabolites.

Oral fluid samples may be collected in a number of ways in order to test for the presence of analytes. One type of sample collector typically includes an absorbent pad for absorbing the target fluid and a holder for holding the sample as the sample is being collected. Once the sample is absorbed by the absorbent pad, the entire pad is transferred to a vial. The vial is then delivered for testing. Disadvantageously, these systems still require additional manipulation, such as centrifugation of the sample in the vial, before the sample can be tested. Other types of sample collectors may release, or express, the sample from the absorbent pad into the vial, rather than placing the entire pad in the vial. Alternatively, the sample may be introduced directly into a testing device, such as a lateral test flow device, ratherthan storing the sample in a vial for subsequent testing. In particular, with typical devices, a precise quantity of oral fluid is not delivered. A metered quantity of oral fluid is critical to ensure that the quantity is sufficient for testing purposes and to allow determination of actual oral fluid concentrations when the oral fluid is combined with a preservative solution.

US 5,103,836 A discloses a method and a device for collecting analytes from the oral cavity for testing. The device is a treated absorbent pad used to collect a specimen having a high concentration of immunoglobulins or other analytes.

CN 101528763 A relates to a device and a method to capture and analyze a sample comprising polynucleotides.

CN 101039683 A discloses compositions and devices including hyaluronic acid and a compound that inhibits degradation of hyaluronic acid.

US2004082878 A1 discloses an oral fluid collection and transfer device comprising a collection device and a test cartridge. The collection device includes a frame or chassis, and an absorbing pad for absorbing oral fluid and which is secured around part of the frame with part of the frame protruding from the pad. The device also includes a fluid adequacy indicatorin the form of an electrical circuit with an LED which is completed when the absorbing means has absorbed a predetermined volume of oral fluid.

US 2008/0118397 A1 discloses a specimen sample collection device including a handle having a sufficiency indicator and an absorbent pad partially contained within said handle.

### SUMMARY

Example embodiments of the claimed invention provide a method and kit for improved collection and expression of oral fluids. For example, a number of embodiments enable users to manipulate the collection device to release an appropriate volume of sample fluid, which can be tested. Moreover, embodiments facilitate sample processing at the testing site of the sample fluid, which is stored and delivered in a vial. For instance, centrifugation can be eliminated as a necessary processing step.

One example embodiment of the claimed invention is an oral fluid sample collection kit that includes a sample collector, a polycarbonate container, and a preservative solution in the polycarbonate container. The sample collector includes an absorbent pad with a collecting element and an interior portion and a handle. The handle includes an upper casing and a lower casing. The absorbent pad extends from the collecting element into the handle. The polycarbonate container receives the absorbent pad or the sample collector itself and holds the pad or collector until testing occurs. The preservative solution in the container inhibits enzymatic activity on a collected sample that can otherwise destroy analytes of interest or otherwise alter the concentration of the analytes for which testing is being conducted. The preservative solution can be a dextran sulfate preservative solution, for example, that inhibits enzymatic activity on the collected sample.

One example embodiment of the claimed oral fluid sample collection kit includes a handle with a sample adequacy window. The sample adequacy window is positioned in the handle and provides visual access to the absorbent pad, which extends beyond the sample adequacy window. The sample adequacy window provides a visual indication as to the sufficiency of the amount of oral fluid sample that is collected. Additionally, the interior portion of the absorbent pad can include an indicator dye positioned between the junction of the collecting element and the interior portion and the sample adequacy window in the handle.

An example embodiment of the claimed invention can include a handle that includes internal pins positioned to fit and secure the upper casing and the lower casing together to form the handle. Additionally, the handle can include textured ridges arranged on the upper casing and/or the lower casing to provide secure handling of the sample collector.

In one embodiment, the collecting element of the absorbent pad can be treated with a hypertonic salt solution including 80 to 170 mg/mL NaCl and 14.3 mg/mL citrate buffer as well as a flavorant or sweetener.

The oral fluid sample collection kit of the claimed invention can also include a preservative solution in the container. The preservative solution can include an anti-microbial agent, an antibacterial agent, an anti-fungal agent, a bacteriostatic agent, a fungistatic agent, an enzyme inhibitor, and combinations of these agents.

In addition to the claimed sample collecting kit, one embodiment of the invention includes a method of collecting an oral fluid sample. The method of the claimed invention includes receiving an oral fluid sample with a sample collector, receiving a dextran sulfate preservative solution in a polycarbonate container, and storing the sample collector in the polycarbonate container with the dextran sulfate preservative solution. The method of collecting the oral fluid sample can include receiving the oral fluid sample with an absorbent pad of the sample collector.

In addition, one embodiment of the claimed invention includes a method of collecting an oral fluid sample that includes viewing an amount of the received oral fluid sample collected through a sample adequacy window in the sample collector. By viewing the amount of sample collected, a user is able to determine sample adequacy volume, that is, whether the volume of sample collected is adequate for testing. Also, the method of collecting an oral fluid sample can include viewing an indicator dye in the amount of the collected oral fluid sample to determine the sample adequacy volume.

In one embodiment, the method of collecting an oral fluid sample includes using a collecting element treated with a hypertonic salt solution including 80 to 170 mg/mL NaCl and 14.3 mg/mL citrate buffer. A flavorant or sweetener can be added to the collecting element as well.

Additionally, in one embodiment of the claimed invention, the method of collecting an oral fluid sample can include using a preservative solution that includes at least one of an anti-microbial agent, an antibacterial agent, an anti-fungal agent, a bacteriostatic agent, a fungistatic agent, and an enzyme inhibitor.

### BRIEF DESCRIPTION OF THE DRAWINGS

Fig. 1 is an exploded view of a sample collector showing an absorbent pad, an upper casing, and a lower casing.
Figs. 2A and 2B are top and bottom views, respectively, of a sample collector showing a collecting element of an absorbent pad and an upper casing with a sample adequacy indicator window.
Fig. 3 is a side view of a sample collector of the claimed invention showing a collecting element of an absorbent pad, an upper casing, and a lower casing.
Fig. 4 is a graph showing increased stability of drugs in a preservative solution of a collecting element.

### DETAILED DESCRIPTION

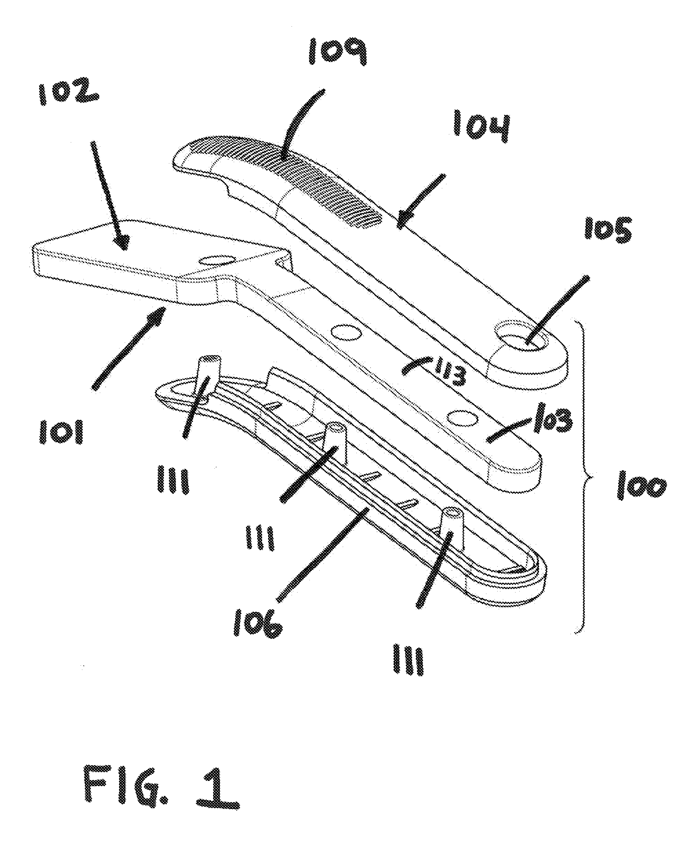

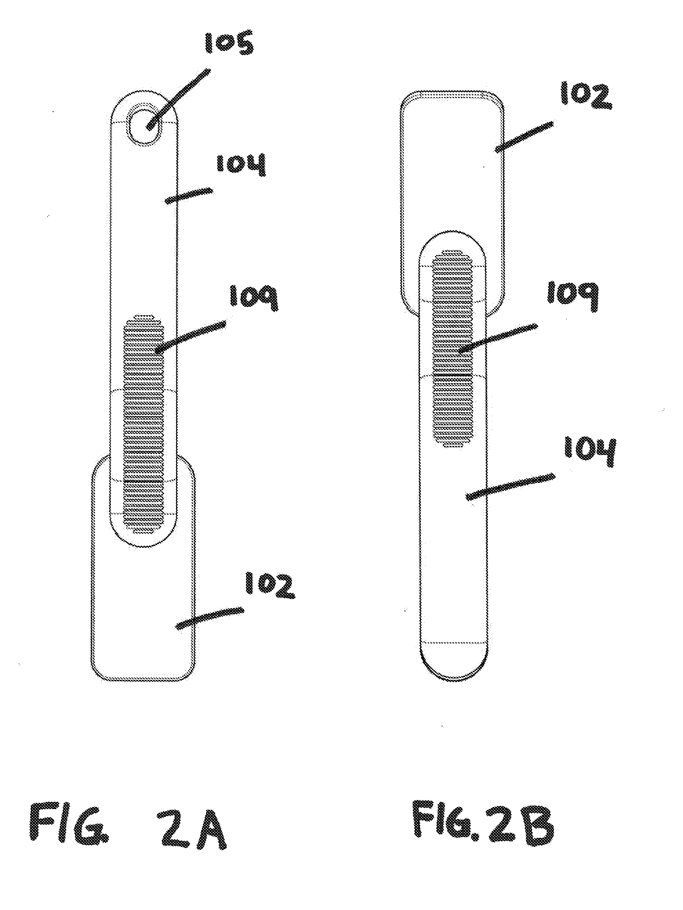

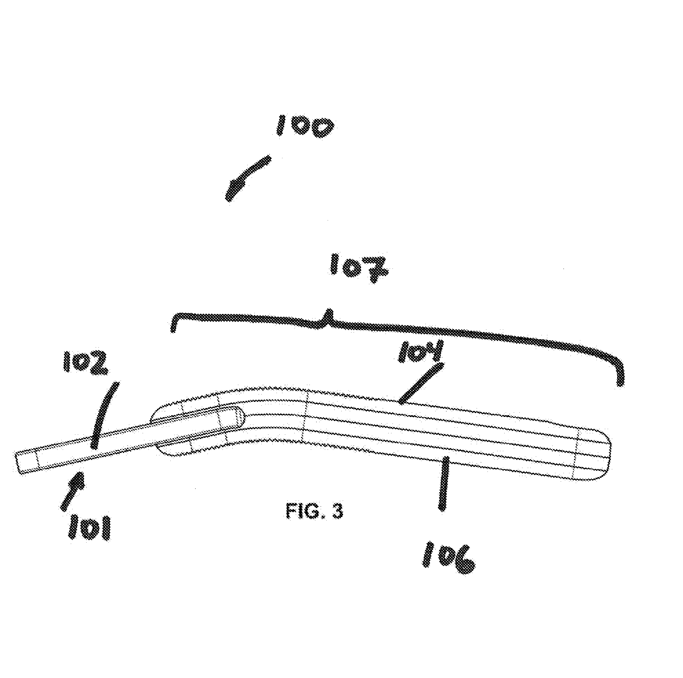

The claimed invention relates to a sample collector 100 for collecting oral fluid samples as shown in Figs. 1-3. A sample collector 100 has an absorbent pad 101, which has a collecting element 102 and an interior portion 103. The absorbent pad 101 can include an absorbent material including natural occurring absorbent materials such as cotton or cellulose materials as well as synthetic absorbent materials such as, but not limited to, polyesters. As shown in Figs. 1 and 3, the absorbent pad 101 has a generally flat profile with a collecting element 102, which is shaped to absorb oral fluid when placed within an oral cavity. The collecting element 102 can be any shape such as an oval, a circle, a square, or as shown in Fig. 1, a rectangle. The collecting element 102 can be sized to collect a desired volume of oral fluid. Generally, about 1 mL is collected. When the collecting element 102 is placed into or in contact with the oral cavity of a subject, it absorbs some of the oral fluid from that source. The absorbent pad 101 also had an interior portion 103 connected to the collecting element 102. The interior portion 103 is dimensioned to extend into a handle 107 (shown in Fig. 3) of a sample collector 100 of the invention, which is formed by the upper casing 104 and the lower casing 106. The interior portion 103 extends in the handle 107 past the sample adequacy window 105.

The upper casing 104 and the lower casing 106 are plastic casings which fit together to form the handle 107 of a sample collector of the invention. The handle 107 surrounds the interior portion 103 of the absorbent pad 101. The casings 104 and 106 form a seal when fitted together to form the handle 107. Casings 104 and 106 may be adhered together using a variety of techniques including ultrasonic welding, gluing, and the like. As shown in Fig. 1, the casings 104, 106 can have internal pins 111 to facilitate fitting the upper casing 104 and the lower casing 106 together. Interior portion 103 of the absorbent pad 101 can be shaped to accommodate the fitting mechanism (such as the internal pins 111) of the casings 104, 106. The handle 107 of a sample collector 100 of the claimed invention can be textured to improve handling as indicated by the ridges 109. The sample collector 100 may be straight or angled, as shown in Figs. 1 and 3.

The upper casing 104 has a sample adequacy window 105 positioned toward the end of the upper casing 104 opposite the collecting element 102. As mentioned above, the interior portion 103 extends in the handle 107 past the sample adequacy window 105. The interior portion 103 is treated with an indicator dye, such as Flag Blue Dye, downstream from the junction of the collecting element 102 and the interior portion 103 but upstream of the area of the interior portion 103 which is seen through the sample adequacy window 105. For example, an indicator dye may be placed about 15 mm upstream from the end of the interior portion 103 at dye placement area 113 as shown in Fig. 1. The dye is placed on the interior portion 103 of the absorbent pad 101 at area 113 and dried before inserting the absorbent pad 101 into the casings 104 and 106 and sealing the device (sample collector 100).

The collecting element 102 can be treated to stimulate oral fluid production and/orto increase the absorbency. For example, absorbent pads treated with a hypertonic salt solution are described in U.S. Patent 5,103,836. To treat the portion of the absorbent pad 101, in one example embodiment, collecting element 102 is allowed to absorb a sufficient amount, approximately 150uL, of a solution containing 80 to 170 mg/mL (for example, 167mg/mL) NaCl and 14.3mg/mL citrate buffer (pH 6.0). Collecting element 102 is then dried. If the treatment causes an unpleasant taste, a flavorant or sweetener or the like can be added to mask the unpleasant taste. Additionally, buffering agents and other agents used to treat oral fluid samples can be dried onto the collecting element 102. The collecting element 102 of the absorbent pad 101 is treated with the solution and then dried before inserting the absorbent pad 101 into the casings 104 and 106 and sealing the device (sample collector 100).

In operation, a user holds a sample collector 100 of the invention by the handle 107 and maneuvers the collecting element 102 of the absorbent pad 101 into or into contact with the subject's oral cavity. The collecting element 102 can be inserted in those areas where oral fluid is excreted and/or collects in the oral cavity. Preferably, the collecting element 102 is placed under the subject's tongue and allowed to collect oral fluid while the device is stationary or the device is moved around the mouth to facilitate the collection. For example, the collecting element 102 may be applied or swabbed inside the mouth, in contact with the gums, to receive a sample of saliva. In another embodiment, the collecting pad 102 is placed inside the mouth between the lower gum and cheek and gently rubbed back and forth along the gum line. In particular, once the collecting element 102 of the absorbent pad 101 comes into contact with an oral fluid source, some of the oral fluid or saliva is drawn, or absorbed, into the absorbent pad 101. The collecting element 102 is left in contact with the oral fluid for a time sufficient to absorb enough oral fluid to fill the absorbent pad 101. As the oral fluid is drawn into and flows into, or wicks up the absorbent pad 101, the fluid encounters an indicator dye dried into the interior portion 103 of the absorbent pad 101 at dye placement area 113. In order to collect a sufficient volume of the sample fluid, the collecting element 102 remains in contact with the oral fluid source until the indicator dye is seen in the sample adequacy window 105. The collecting element 102 may have to remain in contact with the oral fluid source for a specified amount of time. In one example embodiment, the collecting element is placed in contact with the oral fluid for about 30 seconds to about 6 minutes, preferably between about 2 and about 5 minutes. The sample collector 100 is preferably stored in a sealed plastic packaging sleeve and removed just prior to use.

As described previously, samples collected by a sample collector of the invention include saliva, or oral fluid. Accordingly, a further aspect of the invention relates to a method of collecting an oral fluid specimen from an oral cavity for testing. While the method is preferably designed to obtain oral fluid samples to test for drugs of abuse in human subjects, the method can be used to obtain oral fluid sample from humans for other purposes or to obtain oral fluid samples from animals. Once the oral fluid sample is collected, the collecting element 102 is removed from the oral cavity. The fluid sample can then be released, or expressed, from the absorbent pad 101 into a container holding a preservative in a manner employing the systems and devices described above. Alternatively, the collecting element 102 of the sample collector 100 itself can be placed in a preservative solution for later testing of the oral fluid. Thus, it is understood that while the treatments described herein may be employed with the systems and devices described previously, they may be applied more broadly to any system or device for collecting samples of oral fluid.

The oral fluid sample can be expressed from the collection device (sample collector 100) by compressing or squeezing the absorbent pad 101 or by centrifuging the absorbent pad 101. The expressed oral fluid sample can then be analyzed for an analyte of interest. As an alternative to expressing and then analyzing the oral fluid sample, the collecting element 102 containing the oral fluid or the expressed oral fluid sample can also be preserved in a preservative solution for later analysis, as previously described.

A preservative solution, e.g., a preservative solution containing a buffer, surfactant and a salt, can be used with the sample collector of the claimed invention. A preservative solution acts to inhibit enzymatic activity, which can be responsible for the destruction of analytes of interest or can function as an anti-microbial agent. Compounds contemplated for use as a preservative also include antibacterial agents, anti-fungal agents, bacteriostatic agents, fungistatic agents, enzyme inhibitors, and the like.

Table 1 below shows the components and concentration of each component in a preservative used in conjunction with the sample collector 100.

**Table 1**

| **Preservative Solution** | |
|---|---|
| **Component** | **Amount for 1L (g)** |
| Citric Acid | 0.15 |
| Sodium Citrate Dihydrate | 2.95 |
| Dextran Sulfate Sodium Salt | 0.08 |
| Sodium Benzoate | 2.25 |
| Potassium Sorbate | 2.25 |
| Sodium Chloride | 27.2 |
| ProClin 950 Preservative¹ | 1.05 |
| Tween 20 Nonionic Detergent² | 1 |

| | |
|---|---|
| 1. Available from SAFC Supply Solutions, St. Louis, Mo. 2. Available from Sigma Aldrich, St. Louis, Mo. | |

As described previously, samples collected by a sample collector of the invention include saliva, or oral fluid. Accordingly, a further aspect of the invention relates to a method of collecting an oral fluid specimen from an oral cavity for testing. While the method is preferably designed to obtain oral fluid samples to test for drugs of abuse in human subjects, the method may be used to obtain oral fluid sample from humans for other purposes or to obtain oral fluid samples from animals. Once the oral fluid sample is collected, the collecting element 102 is removed from the oral cavity. The fluid sample can then be released, or expressed, from the absorbent material of the absorbent pad 101 into a container containing a preservative in a manner employing the systems and devices described previously. This container and lid containing a preservative is made of polycarbonate plastic Lexan 144R or an equivalent.

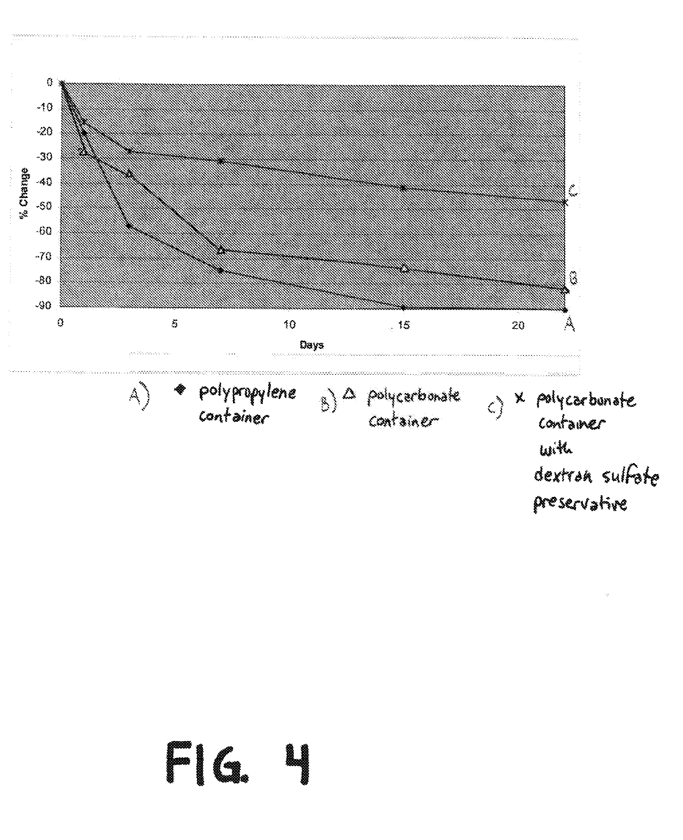

The use of the polycarbonate container and lid and of dextran sulphate as a preservative increases the stability of various drugs in the preservative solution. The graph shown as Fig. 4 illustrates this increased stability for THC when samples are stored at 37°C. Prior devices made of polypropylene contain no dextran sulphate in the preservative solution. This graph of Fig. 4 shows the percent change in sample THC concentration for the sample collection device of the claimed invention in a polypropylene container (filled diamond data points along line A) versus sample THC concentration for a polycarbonate container (open triangle data points along line B) versus sample THC concentration for a polycarbonate container with dextran sulfate preservative (x data points along line C). As shown in the graph, the greatest benefit in maintaining sample concentration is found in using the polycarbonate container with dextran sulfate preservative in accordance with the claimed invention.

That is, the polypropylene container (line A) shows the fastest loss in THC. By changing the polypropylene container to polycarbonate there was a slight increase in THC stability at 15 days from 90% loss to -72% loss. However, the further addition of dextran sulphate to the polycarbonate sample collection device further increased the stability to only a 41% loss at 15 days.

Oral fluid samples collected according to the invention are used in testing for drugs of abuse. For example, the oral fluid samples can be used to test for marijuana (THC), nicotine (continine), cocaine metabolite (benzoylecgonine), opiates (morphine, 6-acetylmorphine, and codeine), phencyclidine, amphetamines (amphetamine and methamphetamine) and other drugs. A variety of assays and testing methods for such drugs of abuse using oral fluid samples can be used. See, for example, E. J. Cone et al., "Oral Fluid Testing for Drugs of Abuse: Positive Prevalence Rates by Intercept Immunoassay Screening and GC-MS-MS Confirmation and Suggested Cutoff Concentrations," J. Analytical. Toxicology, vol. 26, p. 542-6, 2002*.*

## Claims

1. An oral fluid sample collection kit comprising:
a sample collector (100) including
an absorbent pad (101) with a collecting element (102) and an interior portion (103);
a handle (107) including an upper casing (104) and a lower casing (106), the absorbent pad (101) extending into the handle (107);
a polycarbonate container that receives the absorbent pad (101); **characterized in that**
a dextran sulfate preservative solution is included in the polycarbonate container that inhibits enzymatic activity on a collected sample;
the handle (107) also including a sample adequacy window (105) positioned in the handle (107) and providing visual access to the absorbent pad (101) which extends beyond the sample adequacy window (105); and
the interior portion (103) of the absorbent pad (101) also including an indicator dye positioned between the junction of the collecting element (102) and the interior portion (103) and the sample adequacy window (105) in the handle (107).

2. The oral fluid sample collection kit of claim 1, wherein the handle (107) further comprises:
internal pins (111) positioned to fit and secure the upper casing (104) and the lower casing (106) together.

3. The oral fluid sample collection kit of claim 1, wherein the handle (107) further comprises:
textured ridges (109) arranged on at least one of the upper casing (104) and lower casing (106) to provide secure handling of the sample collector (100).

4. The oral fluid sample collection kit of claim 1, wherein the collecting element (102) includes a hypertonic salt solution including 80 to 170 mg/mL NaCl and 14.3 mg/mL citrate buffer.

5. The oral fluid sample collection kit of claim 4, wherein the collecting element (102) includes a flavorant or sweetener.

6. The oral fluid sample collection kit of claim 1, wherein the preservative solution includes at least one of an anti-microbial agent, an antibacterial agent, an anti-fungal agent, a bacteriostatic agent, a fungistatic agent, and an enzyme inhibitor.

7. A method of collecting an oral fluid sample comprising:
Receiving an oral fluid sample with a sample collector (100), sample collector (100) including an absorbent pad (101) with a collecting element (102) and an interior portion (103) and a handle (107) including an upper casing (104) and a lower casing (106), the absorbent pad (101) extending into the handle (107);
receiving a dextran sulfate preservative solution in a polycarbonate container; and
storing the sample collector (100) in the polycarbonate container;
**characterized in that** the polycarbonate container includes a dextran sulfate preservative solution and the method further comprising viewing an indicator dye in an amount of the received oral fluid sample collected through a sample adequacy window (105) in the sample collector (100) to determine sample adequacy volume.

8. The method of collecting an oral fluid sample of claim 7, wherein receiving the oral fluid sample includes receiving the oral fluid sample with the absorbent pad (101) of the sample collector (100).

9. The method of collecting an oral fluid sample of claim 7, wherein the collecting element (102) of the sample collector (100) is treated with a hypertonic salt solution including 80 to 170 mg/mL NaCl and 14.3 mg/mL citrate buffer.

10. The method of collecting an oral fluid sample of claim 7, wherein the collecting element (102) includes a flavorant or sweetener.

11. The method of collecting an oral fluid sample of claim 7, wherein the preservative solution includes at least one of an anti-microbial agent, an antibacterial agent, an anti-fungal agent, a bacteriostatic agent, a fungistatic agent, and an enzyme inhibitor.

## Patentansprüche

1. Probensammelkit für orale Flüssigkeit umfassend:
einen Probensammler (100) enthaltend ein Saugkissen (101) mit einem Sammelelement (102) und einem inneren Abschnitt (103);
einen Griff (107) enthaltend ein oberes Gehäuse (104) und ein unteres Gehäuse (106), wobei sich das Saugkissen (101) in den Griff (107) hinein erstreckt; einen Polycarbonatbehälter, welcher das Saugkissen (101) aufnimmt; **dadurch gekennzeichnet, dass** eine Dextransulfat-Konservierungslösung in dem Polycarbonatbehälter enthalten ist, welche enzymatische Aktivität in einer gesammelten Probe hemmt; der Griff (107) ferner ein im Griff (107) angeordnetes Probe-ausreichend-Fenster (105) enthält, welches visuellen Zugang zu dem Saugkissen (101) erlaubt, welches sich über das Probe-ausreichend-Fenster (105) hinaus erstreckt; und der innere Abschnitt (103) des Saugkissens (101) ferner einen zwischen der Verbindungsstelle des Sammelelements (102) und dem inneren Abschnitt (103) und dem Probe-ausreichend-Fenster (105) im Griff (107) angeordneten Indikatorfarbstoff enthält.

2. Das Probensammelkit für orale Flüssigkeit nach Anspruch 1, wobei der Griff (107) weiterhin umfasst: eingebaute Stifte (111) welche angeordnet sind, um das obere Gehäuse (104) und das untere Gehäuse (106) miteinander zu verbinden und zu befestigen.

3. Das Probensammelkit für orale Flüssigkeit nach Anspruch 1, wobei der Griff (107) weiterhin umfasst: strukturierte Kanten (109) die an wenigstens einem des oberen Gehäuses (104) und des unteren Gehäuses (106) angeordnet sind, um eine sichere Handhabung des Probensammlers (100) zu ermöglichen.

4. Das Probensammelkit für orale Flüssigkeit nach Anspruch 1, wobei das Sammelelement (102) eine hypertonische Salzlösung enthaltend 80 bis 170 mg/mL NaCl und 14,3 mg/mL Citratpuffer enthält.

5. Das Probensammelkit für orale Flüssigkeit nach Anspruch 4, wobei das Sammelelement (102) einen Geschmacksstoff oder einen Süßstoff enthält.

6. Das Probensammelkit für orale Flüssigkeit nach Anspruch 1, wobei die Konservierungslösung zumindest eines von einem antimikrobiellen Mittel, einem antibakteriellen Mittel, einem anti-Pilz-Mittel, einem bakteriostatischen Mittel, einem fungistatischen Mittel, und einem Enzymhemmer enthält.

7. Ein Verfahren zum Sammeln einer Probe einer oralen Flüssigkeit umfassend:
Aufnehmen einer Probe einer oralen Flüssigkeit mit einem Probensammler (100), wobei der Probensammler (100) ein Saugkissen (101) mit einem Sammelelement (102) und einem inneren Abschnitt (103) und einen Griff (107) enthaltend ein oberes Gehäuse (104) und ein unteres Gehäuse (106) enthält, wobei sich das Saugkissen (101) in den Griff (107) hinein erstreckt;
Aufnehmen einer Dextransulfat-Konservierungslösung in einem Polycarbonatbehälter; und
Lagern des Probensammlers (100) in dem Polycarbonatbehälter; **dadurch gekennzeichnet, dass** der Polycarbonatbehälter eine Dextransulfat-Konservierungslösung enthält und das Verfahren weiterhin das Sichten eines Indikatorfarbstoffs in einer Menge der aufgenommenen gesammelten Probe der oralen Flüssigkeit durch ein Probe-ausreichend-Fenster (105) in dem Probensammler (100) umfasst, um ein Ausreichen der Probenmenge zu bestimmen.

8. Das Verfahren zum Sammeln einer Probe einer oralen Flüssigkeit nach Anspruch 7, wobei das Aufnehmen der Probe einer oralen Flüssigkeit das Aufnehmen der Probe der oralen Flüssigkeit mit dem Saugkissen (101) des Probensammlers (100) beinhaltet.

9. Das Verfahren zum Sammeln einer Probe einer oralen Flüssigkeit nach Anspruch 7, wobei das Sammelelement (102) des Probensammlers (100) mit einer hypertonischen Salzlösung enthaltend 80 bis 170 mg/mL NaCl und 14,3 mg/mL Citratpuffer behandelt ist.

10. Das Verfahren zum Sammeln einer Probe einer oralen Flüssigkeit nach Anspruch 7, wobei das Sammelelement (102) einen Geschmacksstoff oder einen Süßstoff enthält.

11. Das Verfahren zum Sammeln einer Probe einer oralen Flüssigkeit nach Anspruch 7, wobei die Konservierungslösung zumindest eines von einem antimikrobiellen Mittel, einem antibakteriellen Mittel, einem anti-Pilz-Mittel, einem bakteriostatischen Mittel, einem fungistatischen Mittel, und einem Enzymhemmer enthält.

## Revendications

1. Ensemble de collecte d'échantillon de fluide buccal comprenant :
un collecteur d'échantillon (100) comprenant
un tampon absorbant (101) avec un élément de collecte (102) et une partie d'intérieur (103) ;
un manche (107) comprenant un boîtier supérieur (104) et un boîtier inférieur (106), le tampon absorbant (101) s'étendant dans le manche (107) ;
un récipient en polycarbonate qui reçoit le tampon absorbant (101) ; **caractérisé en ce qu'**une solution de conservateur de sulfate de dextrane est incluse dans le récipient en polycarbonate qui inhibe l'activité enzymatique sur un échantillon collecté ;
le manche (107) comprenant en outre une fenêtre de capacité d'échantillon (105) positionnée dans le manche (107) et permettant un accès visuel au tampon absorbant (101) qui s'étend au-delà de la fenêtre de capacité d'échantillon (105) ; et
la partie d'intérieur (103) du tampon absorbant (101) comprenant en outre un colorant indicateur positionné entre la jonction de l'élément de collecte (102) et la partie d'intérieur (103) et la fenêtre de capacité d'échantillon (105) dans le manche (107).

2. Ensemble de collecte d'échantillon de fluide buccal de la revendication 1, dans lequel le manche (107) comprend en outre : des broches internes (111) positionnées de manière à s'ajuster et fixer le boîtier supérieur (104) et le boîtier inférieur (106) conjointement.

3. Ensemble de collecte d'échantillon de fluide buccal de la revendication 1, dans lequel le manche (107) comprend en outre : des arêtes texturées (109) agencées sur au moins l'un du boîtier supérieur (104) et du boîtier inférieur (106) pour permettre la manipulation sûre du collecteur d'échantillon (100).

4. Ensemble de collecte d'échantillon de fluide buccal de la revendication 1, dans lequel l'élément de collecte (102) comprend une solution saline hypertonique comprenant 80 à 170 mg/ml de NaCl et 14,3 mg/ml de tampon citrate.

5. Ensemble de collecte d'échantillon de fluide buccal de la revendication 4, dans lequel l'élément de collecte (102) comprend un agent aromatisant ou un édulcorant.

6. Ensemble de collecte d'échantillon de fluide buccal de la revendication 1, dans lequel la solution de conservateur comprend au moins l'un d'un agent antimicrobien, un agent antibactérien, un agent antifongique, un agent bactériostatique, un agent fongistatique, et un inhibiteur d'enzyme.

7. Procédé de collecte d'un échantillon de fluide buccal comprenant :
la réception d'un échantillon de fluide buccal avec un collecteur d'échantillon (100), le collecteur d'échantillon (100) comprenant un tampon absorbant (101) avec un élément de collecte (102) et une partie intérieure (103) et un manche (107) comprenant un boîtier supérieur (104) et un boîtier inférieur (106), le tampon absorbant (101) s'étendant dans le manche (107) ;
la réception d'une solution de conservateur de sulfate de dextrane dans un récipient en polycarbonate ; et
le stockage du collecteur d'échantillon (100) dans le récipient en polycarbonate ;
**caractérisé en ce que** le récipient en polycarbonate comprend une solution de conservateur de sulfate de dextrane et le procédé comprenant en outre la visualisation d'un colorant indicateur dans une quantité de l'échantillon de fluide buccal reçue collectée par l'intermédiaire d'une fenêtre de capacité d'échantillon (105) dans le collecteur d'échantillon (100) pour déterminer le volume de capacité d'échantillon.

8. Procédé de collecte d'un échantillon de fluide buccal de la revendication 7, dans lequel la réception de l'échantillon de fluide buccal comprend la réception de l'échantillon de fluide buccal avec le tampon absorbant (101) du collecteur d'échantillon (100).

9. Procédé de collecte d'un échantillon de fluide buccal de la revendication 7, dans lequel l'élément de collecte (102) du collecteur d'échantillon (100) est traité avec une solution de sel hypertonique comprenant 80 à 170 mg/ml de NaCl et 14,3 mg/ml de tampon citrate.

10. Procédé de collecte d'un échantillon de fluide buccal de la revendication 7, dans lequel l'élément de collecte (102) comprend un arôme ou un édulcorant.

11. Procédé de collecte d'un échantillon de fluide buccal de la revendication 7, dans lequel la solution de conservateur comprend au moins l'un d'un agent antimicrobien, un agent antibactérien, un agent antifongique, un agent bactériostatique, un agent fongistatique, et un inhibiteur d'enzyme.
